# EUROPÄISCHE PATENTSCHRIFT

(11) **EP 0 530 148 B1**
(45) Veröffentlichungstag und Bekanntmachung des Hinweises auf die Patenterteilung: **23.12.1998**
(21) Anmeldenummer: 92810640.0
(22) Anmeldetag: 21.08.1992
(51) Int. Cl.: G03F 7/022, C07C 39/15

(54) **Positiv-Fotoresists mit erhöhtem Auflösungsvermögen und geringer Kristallisationsneigung, sowie neue Tetra(hydroxyphenyl)alkane**
Positive photo resist with increased dissolving power and reduced crystallisation tendency as well as new tetra(hydroxyphenyl)alkane
Photoréserve positive ayant une plus grande solubilité et plus faible tendance à cristalliser, ainsi que des nouveaux tetra(hydroxyphényl)alkanes

(30) Priorität: 30.08.1991 CH 2550/91; 12.02.1992 CH 413/92
(43) Veröffentlichungstag der Anmeldung: 03.03.1993
(73) Patentinhaber: Olin Microelectronic Chemicals, Inc., Norwalk, Connecticut 06856-4500 (US)
(72) Erfinder: Schulz, Reinhard, Dr., W-7813 Staufen-Wettelbrunn (DE); Münzel, Norbert, Dr., W-7843 Heitersheim (DE); Roth, Martin, Dr., CH-1735 Giffers (CH); Knobloch, Wilhelm, Dr., W-7881 Schwörstadt (DE)
(74) Vertreter: Klunker . Schmitt-Nilson . Hirsch

(56) Entgegenhaltungen:
- EP-A- 0 416 544
- EP-A- 0 443 820
- PATENT ABSTRACTS OF JAPAN vol. 9, no. 276 (P-402)(1999) 2. November 1985 & JP-A-60 121 445 ( NIPPON GOSEI GOMU K.K. ) 28. Juni 1985

## Beschreibung

Die Erfindung betrifft Positiv-Fotoresist-Zusammensetzungen, die ein erhöhtes Auflösungsvermögen und eine geringe Kristallisationsneigung aufweisen, wobei als Verbindung zur Steigerung der Fotoempfindlichkeit (Speed-enhancer) bestimmte, z.T. neue Tetra(p-hydroxyphenyl)alkane eingesetzt werden.

Als Fotoresists werden üblicherweise fotostrukturierbare organische Polymermaterialien bezeichnet, die in fotolithographischen Prozessen und verwandten Techniken eingesetzt werden, beispielsweise bei der Herstellung von Druckplatten, von gedruckten elektrischen Schaltungen und Leiterplatten oder insbesondere in der Mikroelektronik bei der Herstellung von integrierten Halbleiterbauelementen.

Zur Erzeugung der Schaltungsstrukturen bei der Herstellung von integrierten mikroelektronischen Halbleiterbauelementen wird das Halbleitersubstratmaterial mit dem Fotoresist beschichtet; durch bildmässiges Belichten der Fotoresistschicht und anschliessendes Entwickeln werden dann positive oder negative Fotoresistreliefstrukturen erhalten. Diese dienen als Maske für die eigentlichen Strukturierungsprozesse am Halbleitersubstrat wie Aetzen, Dotieren, Beschichten mit Metallen, anderen Halbleiter- oder auch Isolatormaterialien. Danach werden die Fotoresistmasken in der Regel wieder entfernt. Durch eine Vielzahl derartiger Prozesszyklen entstehen auf dem Substrat die Schaltungsstrukturen der Mikrochips.

Positiv-Fotoresists üblicher Art enthalten in einem organischen Lösungsmittel im wesentlichen mindestens je ein in wässrigen Alkalien lösliches Harz und eine fotoempfindliche Chinondiazidverbindung, die die Alkalilöslichkeit des Harzes reduziert, sowie üblicherweise noch weitere modifizierende Zusätze. Durch Strahlungseinwirkung auf mit derartigen Zusammensetzungen erzeugte Fotoresistschichten wird die Alkalilöslichkeit in den belichteten Bereichen durch fotoinduzierte strukturelle Umwandlung der Chinondiazidverbindung in ein Carbonsäurederivat erhöht, so dass nach Entwicklung in wässrig-alkalischen Entwicklungsbädern positive Fotoresist-Reliefstrukturen erhalten werden.

Unter den anwendungstechnisch wesentlichen Merkmalen eines Fotoresists sind insbesondere die Eigenschaften bezüglich Strahlungsempfindlichkeit, Bildauflösung, Kontrast und Lagerbeständigkeit wichtig.

Eine hohe Empfindlichkeit ist wichtig, um in den produktionstechnischen Prozesszyklen kurze Bestrahlungszeiten zu gewährleisten, auch etwa, wenn gerätetechnisch bedingt nur Strahlung geringerer Intensität auf den Resist einwirken kann.
Die Bildauflösung charakterisiert, bis zu welchen Dimensionen kleinste Bildstrukturen, wie etwa Linien und Zwischenräume, klar getrennt durch den Fotoresist wiedergegeben werden können. Z.B. erfordert die Herstellung von VLSI-Schaltungen die Wiedergabe von Strukturdetails in der Grössenordnung von 1 µm und weniger.
Der Kontrast charakterisiert die Kantensteilheit und Kantenschärfe der nach der Entwicklung erhaltenen Fotoresist-Reliefstrukturen.

Die ständig fortschreitende Miniaturisierung in der Halbleitertechnologie und Mikroelektronik stellt an die Fotoresist-Materialien und die damit darzustellenden Reliefstrukturen höchste Anforderungen, insbesondere hinsichtlich Empfindlichkeit, Auflösung und Kontrast, aber auch an die Haftfestigkeit, mechanische und chemische Stabilität, Dimensionstreue und Beständigkeit bei erhöhten Temperaturen oder anderen Einflüssen, die bei den weiteren Prozessschritten auf die Fotoresist-Reliefstrukturen einwirken können.

Zu diesem Zweck enthalten die gängigen Positiv-Fotoresists neben den Basiskomponenten alkalilösliches Harz und Chinondiazidverbindung meist auch die verschiedensten Zusatzstoffe, um die Eigenschaften des Fotoresists hinsichtlich der genannten Anforderungen zu optimieren. So sind beispielsweise als empfindlichkeitssteigernde Additive aus den US-Patenten 3,661,582 und 3,365,019 Stickstoffheterozyklen-Verbindungen, wie Benzotriazol oder halogenierte Benzotriazol-Derivate, bekannt. Gemäss den US-Patenten 4,036,644 bzw. 4,115,128 werden für den gleichen Zweck aliphatische Verbindungen mit einer oder mehreren Carboxylgruppen bzw. organische zyklische Anhydride eingesetzt. Gemäss der EP-Patentanmeldung 416544 werden als Zusätze Tetrahydroxyphenole vorgeschlagen. Als besonders effektive Zusätze zur Steigerung der Empfindlichkeit und der Entwicklungsrate werden in dem US-Patent 4,626,492 Trihydroxybenzophenone beschrieben, insbesondere die Verbindung 2,3,4-Trihydroxybenzophenon. Aber auch diese Verbindungen vermögen die steigenden Anforderungen nicht immer zu befriedigen. Der Effekt der Steigerung der Entwicklungsrate durch Zusätze, die hydrophile oder saure Gruppen aufweisen, wie es etwa bei polyhydroxylierten Benzolderivaten der Fall ist, beruht im wesentlichen auf einer Erhöhung der Löslichkeit des Resists in wässrig-alkalischen Medien. Der Zusatz von undifferenziert die Löslichkeit steigernden Additiven zum Fotoresist, wie auch die Verwendung von schnelleren Entwicklern höherer Ionenkonzentration, hat jedoch den Nachteil, dass es bei der Entwicklung auch zu einem verstärkten Angriff der unbelichteten Resistbereiche kommt. Hierdurch wird der Dunkelabtrag vergrössert, d.h. die Schichtdicke der verbleibenden Fotoresist-Reliefstrukturen wird vermindert. Des weiteren wird die Differenzierung der Löslichkeit zwischen belichteten und unbelichteten Bereichen schlechter, was zu einer Verringerung des Kontrastes, also einer Verminderung der Kantensteilheit und Kantenschärfe, und damit der Abbildungsqualität führt.

Schliesslich wird gemäss der EP-A-435437 zur Verbesserung der Fotoempfindlichkeit vorgeschlagen, bestimmte hydroxylierte Bis-Diphenylmethan-Verbindungen einzusetzen.

Es wurde nun gefunden, dass der Einsaz von bestimmten Tetra(hydroxyphenyl)alkane in Positiv-Fotoresist-Formulierungen die Fotoempfindlichkeit und/oder die Entwicklungsrate merklich steigert, ohne dass dabei die üblichen Eigenschaften, insbesondere die Abbildungsqualität, beeinträchtigt werden.

Gegenstand vorliegender Erfindung sind somit Positiv-Fotoresist-Zusammensetzungen enthaltend in einem organischen Lösungsmittel mindestens je
a) ein alkalilösliches Harz,
b) eine fotoempfindliche Chinondiazid-Verbindung,
c) eine aromatische Hydroxyverbindung der Formel I 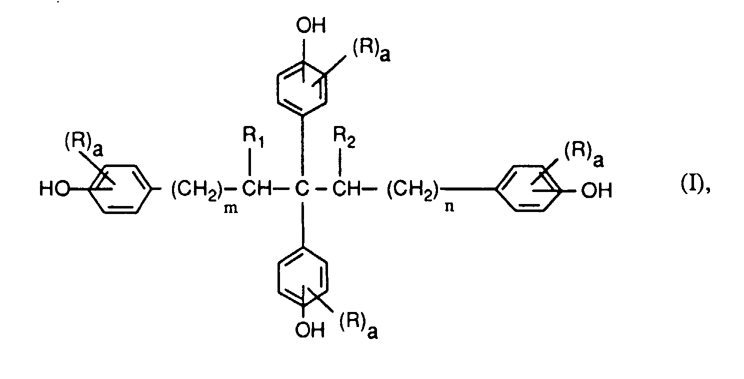 worin R unabhängig voneinander -H, C₁-C₄-Alkyl, C₁-C₄-Alkoxy, -OCH₂C₆H₅, -OC₆H₅ oder -COOC₁-C₄-Alkyl, und R₁ und R₂ unabhängig voneinander H, C₁-C₄-Alkyl, -C₆H₅ oder einen cycloaliphatischen 5- oder 6-Ring bedeuten, a eine ganze Zahl von Null bis 4, und m und n unabhängig voneinander die Zahl Null, 1 oder 2 darstellen,
   sowie gegebenenfalls
d) weitere übliche Zusätze.

C₁-C₄-Alkyl und C₁-C₄-Alkoxy bedeuten z.B. Methyl, Ethyl, n-Propyl, Isopropyl, n-Butyl oder tert.-Butyl bzw. Methoxy, Ethoxy, n-Propoxy, Isopropoxy, n-Butoxy oder tert.-Butoxy.

Erfindungsgemäss bevorzugt sind Verbindungen der Formel I, worin R und R₁ -H bedeuten bzw. a Null ist und m und n die Zahl 1 oder 2 darstellen.

Ganz bevorzugt als Komponente c) sind Verbindungen der Formel II 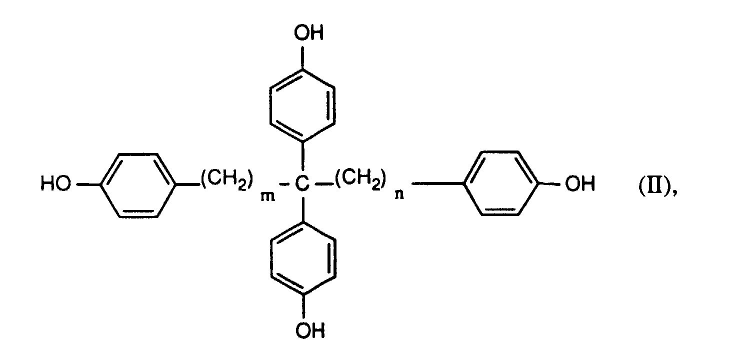 worin m und n unabhängig voneinander die Zahl 1, 2 oder 3 darstellen.

Gegenstand der Erfindung ist weiterhin die Verwendung der Verbindungen der Formel I als Additive zur Steigerung der Fotoempfindlichkeit und/oder der Entwicklungsrate in Positiv-Fotoresist-Zusammensetzungen auf Basis alkalilöslicher Harze und lichtempfindlicher Chinondiazid-Verbindungen.

Die erfindungsgemässen Positiv-Fotoresist-Zusammensetzungen entsprechen bezüglich der Hauptkomponenten im wesentlichen den bekannten und handelsüblichen Positiv-Fotoresists. Sie enthalten in einem organischen Lösungsmittel ein alkalilösliches Harz, eine fotoempfindliche Chinondiazid-Verbindung und gegebenenfalls weitere übliche Zusatzstoffe, mit denen die Eigenschaften des Fotoresist auf die spezifischen Anforderungen der Einsatzzwecke eingestellt werden können.

Die Komponente c) wird zweckmässig in einer Konzentration zwischen 0,5 und 30, bevorzugt zwischen 1 und 20 Gew.%, bezogen auf den Gesamtfeststoffgehalt der Fotoresist-lösung, eingesetzt.

Ganz bevorzugt sind Verbindungen der Formel II, worin m und n 2 sind.

Die erfindungsgemässen Positiv-Fotoresist-Zusammensetzungen enthalten als Harzkomponente a) alkalilösliche Harze, wie z.B. Novolak-Harze, die durch Kondensation von Phenol bzw. phenolischen Verbindungen mit Aldehyden erhalten werden.

Beispiele von phenolischen Verbindungen sind Phenol, Kresole, Xylenole, Ethyl-, Propyl-, Butyl- und Phenylphenole, Resorcin, Pyrocatechol, Hydrochinon, Bisphenol A und Pyrogallol.

Beispiele von Aldehyden sind Formaldehyd, Acetaldehyd, Benzaldehyd und Teraphthalsäure-dialdehyd.

Bevorzugt sind Kresol-Formaldehyd-Harze, zu deren Herstellung o-, m-, p-Kresol oder Mischungen dieser Isomeren in beliebigen oder vorbestimmten Verhältnissen eingesetzt werden. Die Herstellung solcher Harze und ihr Einsatz in Positiv-Fotoresists ist z.B. im US-Patent 4,377,631 beschrieben.

In den erfindungsgemässen Positiv-Fotoresist-Zusammensetzungen ist die Harzkomponente üblicherweise in einem Anteil von etwa 50-95 Gew.%, bezogen auf den Gesamtfeststoffgehalt der Fotoresist-Lösung, enthalten.

Ausserdem kommen auch andere alkalilösliche Harze in Frage, wie sie vielfach in Positiv-Fotoresists verwendet werden. Dazu gehören etwa Polyvinylphenole und Polyglutarimide, Copolymere aus Styrol und α-Methylstyrol mit Maleinimid sowie Copolymere aus N-(p-Hydroxyphenyl)-maleinimid und Olefinen. Weiterhin können auch silylierte alkalilösliche Polymere verwendet werden, die eine höhere Plasmaätzbeständigkeit aufweisen.

Andere geeignete alkalilöschliche Harze leiten sich z.B. von Acrylsäure, Methacrylsäure, Styrol, Maleinsäureanhydrid, Maleinimid, Vinylacetat, Acrylonitril und Derivaten dieser Verbindungen, sowie Gemischen davon zur Herstellung des entsprechenden Copolymeren ab.
Zur Verbesserung ihrer allgemeinen Eigenschaften, wie Lagerbeständigkeit, Entwicklungsrate und/oder thermische Beständigkeit, können den erfindungsgemäss in Frage kommenden Harzen weitere dem Fachmann bekannte Zusatzstoffe beigemischt werden.

Als fotoempfindliche Chinondiazidverbindungen in Positiv-Fotoresists sind Chinondiazidsulfonsäureester enthalten, z.B. Veresterungsprodukte der 2,1-Naphthochinon-1-diazid-4-oder -5-sulfonsäure oder bevorzugt der 1,2-Naphthochinon-2-diazid-4- oder -5-sulfonsäure, mit niedermolekularen aromatischen oder aliphatischen Hydroxyverbindungen. Derartige Naphthochinondiazidsulfonsäureester sind beispielsweise in den US-Patentschriften 3,046,112, 3,106,462, 3,148,983 und 3,201,239 sowie in EP 0085761 beschrieben. Ueberwiegend sind in Positiv-Fotoresists als lichtempfindliche Komponenten Naphthochinondiazidsulfonsäueester von Hydroxybenzophenonen, wie insbesondere 2,3,4-Trihydroxybenzophenon oder 2,3,4,4'-Tetrahydroxybenzophenon im Gebrauch. Vorteilhaft sind auch die entsprechenden Ester mehrfach hydroxylierter Benzole, wie etwa die Isomeren von Trihydroxybenzol, z.B. 1,2,3-, 1,2,4- und insbesondere 1,3,5-Trihydroxybenzol, ferner mehrfach hydroxylierter Biphenyle, wie z.B. 1,3',4,5',6-Pentahydroxybiphenyl. Beispiele derartiger Benzophenone und Biphenyle sind in der EP-A 335836 beschrieben.

Beispiele von Hydroxyverbindungen sind Kresole, Xylenole, Resorcin, Catechol, 2,2'- und 4,4'-Dihydroxybiphenyl, Hydrochinon, Pyrogallol, Phloroglucin, 4,4'-Dihydroxybenzophenon, Bis(4-hydroxyphenyl)ether, Bis(4-hydroxyphenyl)sulfid, Bis(2,4-dihydroxyphenyl)sulfid, Bis(4-hydroxyphenyl)sulfon, 2,3,4-Trihydroxybenzophenon, 2,4,4'-Trihydroxybenzophenon, 2,3,4,4'-Tetrahydroxybenzophenon, 2,2',4,4'-Tetrahydroxybenzophenon, 2,2',3,4,4'-Pentahydroxybenzophenon, 2,2',3,4,5'-Pentahydroxybenzophenon, 2,3,3',4,5-Pentahydroxybenzophenon, 2,3,3',4,4',5-Hexahydroxybenzophenon, 2,3',4,4',5',6-Hexahydroxybenzophenon, Gallussäure-methyl-, Ethyl- und -propylester, 2,2-bis(4-Hydroxyphenyl)propan, 2,2-bis(2,4-Dihydroxyphenyl)propan, 2,2-bis (2,3,4-Trihydroxyphenyl)propan, Kresol-Novolak-Harze, Resorcin-Aceton-Harze, Pyrogallol-Aceton-Harze oder Polyvinylphenolharze. Auch Kombinationen bzw. Gemische der oben aufgeführten Naphthochinondiazidverbindungen können eingesetzt werden.

Von den lichtempfindlichen Naphthochinondiazidverbindungen sind überwiegend die 5-Sulfonssäureester im Gebrauch. Diese verfügen über eine breite Absorption im nahen bis mittleren UV-Wellenlängenbereich mit Maxima bei etwa 400 nm und etwa 340 nm. In diesem Wellenlängenbereich liegen kräftige Emissionslinien der üblicherweise in den Projektionsgeräten eingesetzten Quecksilberlampen, wie etwa die Linien bei 334 nm, 365 nm und 405 nm. Die 4-Sulfonsäureester verfügen über Absorptionsmaxima bei ca. 380 nm und um 300 nm und sind daher mehr für mittlere bis kurzwelligere UV-Strahlung geeignet, beispielsweise die Quecksilberemissionslinie bei 313 nm.

Die Chinondiazidverbindungen sind in Positiv-Fotoresists üblicherweise in einem Anteil von etwa 5-50 Gew.%, bezogen auf den Gesamtfeststoffgehalt der Fotoresist-Lösung, enthalten.

Andere Chinondiazidverbindungen, die auch verwendet werden können, sind zum Beispiel monomere und dimere 2-Diazo-1,3-diketone, α-Phosphoryldiazocarbonylverbindungen sowie Benzochinondiazid-Derivate.

Besonders bevorzugt sind Naphtochinon-Sulfonsäureester aus 2,3,3-Trihydroxybenzophenon, 2,3,4,4'-Tetrahydroxybenzophenon, Bis(4-hydroxyphenyl)sulfon, 4,4'-Dihydroxybenzophenon oder Bis(2,4-dihydroxyphenyl)sulfid und insbesondere 2,3,4-Trihydroxybenzophenon und 2,3,4,4'-Tetrahydroxybenzophenon.

Ganz bevorzugt sind Veresterungsprodukte der 1,2-Naphthochinon-2-diazid-5-sulfonsäure mit einem mehrfach hydroxylierten Benzophenon oder Biphenyl.

Als fotoempfindliche Chinondiazidverbindungen in Positiv-Fotoresist-Formulierungen können auch Veresterungsprodukte der 1,2-Naphthochinon-2-diazid-4-sulfonsäure oder 1,2-Naphthochinon-2-diazid-5-sulfonsäure oder der 2,1-Naphthochinon-1-diazid-4- oder -5-sulfonsäure mit einer mehrfach hydroxylierten Verbindung der oben erwähnten Formel I verwendet werden. Derartige Veresterungsprodukte sind neu und stellen daher auch einen Erfindungsgegenstand dar.

Bevorzugte Veresterungsprodukte sind solche der 1,2-Naphthochinon-2-diazid-5-sulfonsäure mit einer Verbindung der Formel I, worin m und n 1 oder 2 sind.

Derartige Veresterungsprodukte eignen sich ausgezeichnet als fotoaktive Komponente in Positiv-Fotoresist-Formulierungen und zeichnen sich durch gute allgemeine anwendungstechnische Eigenschaften aus, insbesondere durch ein verbessertes Auflösungsvermögen und eine verbesserte Lagerstabilität, sowie eine verminderte Kristallisationstendenz in derartigen Formulierungen.

Die neuen Veresterungsprodukte können nach an und für sich bekannten Verfahren hergestellt werden, indem man ein 2,1-Naphthochinon-1-diazid-4- oder -5-sulfonsäurehalogenid oder ein 1,2-Naphthochinon-2-diazid-4-sulfonsäure- oder -5-sulfonsäurehalogenid, insbesondere ein Säurechlorid, mit einer Verbindung der Formel I in etwa stöchiometrischen Mengen oder im Ueberschuss zweckmässig in einem inerten organischen Lösungsmittel und bei erhöhter Temperatur umsetzt,
oder aber durch Umsetzung eines niedrigen Alkylesters, z.B. eines Methyl- oder Ethylesters, der entsprechenden Naphthochinon-diazid-4- oder -5-sulfonsäure bei erhöhter Temperatur zweckmässig unter gleichzeitigem Abdestillieren des dabei entstandenen niedrigen Alkylalkohols.

Als organische Lösungsmittel sind im Prinzip alle inerten Lösungsmittel geeignet, z.B. aliphatische Ketone, wie Aceton, Methylethylketon oder Methylisobutylketon, ferner Cyclohexanon, aliphatische Ester, wie Ethyl-, Propyl- oder Butylacetat, Ether, wie Dioxan, Diethylether oder Tetrahydrofuran, ferner y-Butyrolacton, aliphatische oder aromatische Kohlenwasserstoffe, wie Hexan, Octan, Chlorbenzol, Dichlorbenzole, Toluol, Xylole oder Nitrobenzol, femer Formamid, Dimethylformamid, N-Methylpyrrolidon, oder Gemische derartiger Lösungsmittel.

Einige Verbindungen der Formel I, nämlich unsubstituierte und durch 3-Chlor, 5-Methyl oder 5-Ethyl substituierte 1,2,2,3-Tetrakis-(2- oder 4-hydroxyphenyl)-propanderivate [R₁, R₂ = H; m und n = Null; a = Null oder 1] sind bekannt und im US-Patent Nr. 2965611 beschrieben. Die übrigen Verbindungen der Formel I dagegen sind neu.
Sie können erhalten werden z.B. durch Kondensation eines Hydroxybenzaldehyds (z.B. des para-Hydroxybenzaldehyds) mit einem aliphatischen Keton, z.B. mit Aceton, zu einem ungesättigten Chalconderivat, das durch regioselektive Reduktion der beiden C,C-Doppelbindungen im Ketonrest und anschliessende Umsetzung des erhaltenen Bis(hydroxyphenyl)ketons, z.B. 1,5-Bis(p-hydroxyphenyl)pentan-3-on, mit einem Ueberschuss an Phenol unter Säurekatalyse zum Tetra(hydroxyphenyl)alkan der Formel I, z.B. 1,3,3,5-Tetra(p-hydroxyphenyl)pentan, umgesetzt wird.

Die benötigten Ausgangsprodukte Bis(hydroxyphenyl)ketone sind z.T. auch bekannt und können nach an und für sich bekannten Verfahren gewonnen werden. Beispiele davon sind z.B. im Chem. Ber. 52 (1919), S. 2078 oder im US-Patent Nr. 4552876 gemäss Bsp. 17 beschrieben.

Die Verbindungen der Formeln I bzw. II stellen wertvolle Ausgangsprodukte dar, welche nicht nur für die oben bereits angegebene Verwendung (Herstellung von Naphtochinondiazidestern und Verwendung als Zusatz in Fotoresists) sondern z.B. auch zur Herstellung von hochvernetzbaren Epoxidharzen durch z.B. Glycidylisierung der (Tetrahydroxylphenyl)alkane der Formel I und anschliessende Härtung eingesetzt werden können. Derartige Tetrahydroxyphenole können auch als Härter für Epoxidharze verwendet werden; die damit gehärteten Produkte zeichnen sich durch eine hohe Glasumwandlungstemperatur (Tg) und gute mechanische Eigenschaften sowie hohe Chemikalienbeständigkeit aus. Ausserdem können diese Polyphenole zur Herstellung von Tetracyanatestern als Harze zur Herstellung von wärmefesten Duroplasten eingesetzt werden oder als Verzweigungsmittel für statistisch verzweigte Polycarbonate gemäss DE-OS 3035204 verwendet werden.

Als Lösungsmittel zur Herstellung der Fotoresist-Lösung sind im Prinzip alle Lösungsmittel geeignet, in denen die festen Fotoresist-Bestandteile, wie alkalilösliches Harz, die Chinondiazidverbindungen und gegebenenfalls weitere Zusatzstoffe, ausreichend löslich sind und die mit diesen Bestandteilen nicht irreversibel reagieren. In Frage kommen hierfür beispielsweise aliphatische Ketone, wie Methylethylketon oder Cyclohexanon, aliphatische Ester, wie Butylacetat, Ether, wie Tetrahydrofuran, Alkohole, wie n- oder i-Propanol, Mono- oder Bisether sowie gemischte Ether-Esterderivate von Glycolverbindungen, wie von Ethylenglycol, Diethylenglycol oder Propylenglycol, des weiteren Monooxocarbonsäureester, wie etwa Milchsäureethylester oder 2-Ethoxypropionsäureethylester. Aliphatische und aromatische Kohlenwasserstoffe, wie n-Hexan und Xylol, finden ebenfalls Verwendung als Lösungsmittel. Vielfach werden auch Gemische aus den genannten Lösungsmitteln verwendet. In vielen Fällen enthalten Fotoresist-Lösungsmittel Ethoxy-ethylacetat, Propylenglycolmethyletheracetat oder Ethylenglycoldimethylether. Das Lösungsmittel hat üblicherweise einen Anteil von 40-95 Gew.% an der gesamten Fotoresist-Lösung.

Zu den weiteren üblichen Zusatzstoffen, die auch in den erfindungsgemässen Fotoresist-Zusammensetzungen enthalten sein können, gehören etwa streustrahlungsabsorbierende Substanzen und Farbstoffe, Verlaufmittel, Weichmacher, Haftvermitter, weitere filmbildende Harze, Entwicklungsbeschleuniger, Tenside und Stabilisatoren. Zusätze dieser Kategorien sind dem Fachmann ausreichend bekannt und sind vielfältig in der einschlägigen Fachliteratur beschrieben. Der Anteil an solchen Zusätzen übersteigt zusammengenommen kaum 25 Gew.%, bezogen auf den Gesamtfeststoffgehalt der Fotoresist-Lösung.

Die erfindungsgemässen Fotoresist-Zusammensetzungen enthalten typischerweise 50-90 Gew.%, vorzugsweise 60-80 Gew.%, an alkalilöslichem Harz, 5-30 Gew.%, vorzugsweise 10-25 Gew.%, an Chinondiazid [Komponente b)], 5-20 Gew.%, an Komponente c), und 0-30 Gew.%, vorzugsweise 0,1-5 Gew.%, an sonstigen Zusatzstoffen, jeweils bezogen auf den Gesamtfeststoffgehalt.

Die Formulierung der erfindungsgemässen Positiv-Fotoresist-Zusammensetzungen erfolgt in an sich bekannter Weise durch Mischen bzw. Lösen der Komponenten in dem Lösungsmittel bzw. Lösungsmittelgemisch, wobei die Komponente c) zugesetzt wird. Gleichermassen ist es möglich, fertigformulierte Positiv-Fotoresist-Zusammensetzungen, wie sie etwa im Handel sind, mit der entsprechenden Menge an Komponente c) zu versetzen. Nach Lösung der Bestandteile im Lösungsmittel wird die erhaltene Fotoresist-Lösung je nach Anspruch an die Partikelfreiheit durch Membranfilter mit einer Porenweite von 0,1-1 µm filtriert. Ueblicherweise wird der Gesamtfeststoffgehalt des Fotoresist auf die gewünschte Schichtdicke und Beschichtungsmethode abgestimmt.

Die Anwendung der erfindungsgemässen Positiv-Fotoresist-Zusammensetzungen erfolgt nach an sich bekannten Verfahren und mit den hierfür üblichen Prozessgeräten. Zunächst wird die Fotoresist-Lösung auf das Substrat aufgebracht und getrocknet. Als Substrate fungieren überwiegend Halbleiterscheiben, wie z.B. Silizium-Wafer, die gegebenenfalls mit einer Schicht aus Siliziumdioxid, Siliziumnitrid oder Aluminium überzogen sind Auch andere, bei der Herstellung miniaturisierter Schaltungen übliche Materialien, wie Germanium, Galliumarsenid, Keramik mit gegebenenfalls Edelmetallbeschichtung, kommen in Frage.

Die Beschichtung erfolgt üblicherweise durch Tauchen, Sprühen, Walzen oder Aufschleudern. Bei der letzteren, am häufigsten angewandten Beschichtungsmethode ist die resultierende Schichtdicke abhängig von der Viskosität der Fotoresist-Lösung, dem Feststoffgehalt und der Aufschleudergeschwindigkeit. Für den jeweiligen Fotoresist werden sogenannte Schleuderkurven ermittelt, aus denen sich die Resist-Schichtdicken in Abhängigkeit von Viskosität und Schleuderdrehzahl ermitteln lassen. Die Schichtdicken bei Positiv-Fotoresists liegen typisch im Bereich von 0,5-4 µm, insbesondere 0,6-2,0 µm.

Nachdem der Fotoresist auf das Substrat aufgebracht worden ist, wird er normalerweise bei Temperaturen zwischen 70°C und 130°C vorgetrocknet. Dazu können Oefen oder Heizplatten (sogenannte "Hot Plates") verwendet werden. Die Trocknungszeit in Oefen liegt im allgemeinen im Bereich von etwa 15-45 Minuten, auf Hot Plates im Bereich von etwa 0,5-4 Minuten. Vorzugsweise werden 0,5-2 µm dicke Resistschichten etwa 1 Minute bei etwa 100°C auf der Hot Plate getrocknet.

Der getrocknete Fotoresist wird dann durch eine Maske hindurch bildmässig belichtet, wobei Strahlung mit einer Wellenlänge im Bereich von etwa 300 nm bis 450 nm verwendet wird. Die Belichtung kann poly- oder monochromatisch erfolgen. Bevorzugt werden hierzu kommerziell erhältliche Geräte, wie z.B. Scanning-Projektions-Belichtungsgeräte, Kontakt- und Abstands-Belichtungsgeräte oder Wafer-Stepper eingesetzt.

Die mit dem Fotoresist beschichteten und belichteten Substrate werden schliesslich mit einer wässrig-alkalischen Entwicklerlösung, z.B. durch Tauchen oder Besprühen, entwickelt, bis in den belichteten Bereichen der Resist vollständig abgelöst ist. Es können verschiedene Entwicklerformulierungen verwendet werden, die entweder zur Klasse der metallionenhaltigen oder metallionenfreien Fotoresistentwickler gehören. Metallionenhaltige Entwickler sind z.B. wässrige Lösungen von Natriumhydroxyd oder Kaliumhydroxid, die darüber hinaus pH-regulierende und puffernde Substanzen, wie Phosphate oder Silikate sowie Tenside und Stabilisatoren enthalten können. Metallionenfreie Entwickler sind wässrige Lösungen von z.B. organischen Basen, wie z.B. Tetramethyl- oder Tetraethylammoniumhydroxyd oder Cholin, ferner primäre, sekundäre oder tertiäre Amine, wie Ethyl-, Propyl-, Diethyl-, Dipropyl-, Trimethyl- und Triethylamin, ferner Aminoalkohole, wie Diethanolamin und Triethanolamin. Die Entwicklungszeiten hängen von der Belichtungsenergie, Stärke des Entwicklers, Art des Entwickelns, der Vortrocknungstemperatur und der Entwicklertemperatur ab. Bei der Tauchentwicklung sind Entwicklungszeiten von etwa 1 Minute typisch. Die Entwicklung wird üblicherweise durch Eintauchen oder Besprühen mit deionisiertem Wasser gestoppt. An die Entwicklung schliesst sich häufig eine Nachtrocknung bei etwa 120°C bis 180°C an.

Die mit den erfindungsgemässen Fotoresist-Zusammensetzungen hergestellten Reliefstrukturen zeigen eine vorzügliche Bildauflösung bis unter 1 µm bei hohem Kontrast, hoher Kantensteilheit und Kantenschärfe. Der Schicktdickenverlust in den unbelichteten Bereichen ist minimal. Ausserdem zeichnen sich die vorliegenden Fotoresist-Zusammensetzungen durch ein verbessertes Auflösungsvermögen und eine geringe Kristallisationsneigung, sowie durch verbesserte Fotoempfindlichkeit, aus. In den nachfolgenden Prozessabläufen bei der Herstellung von integrierten Halbleiterschaltungen, wie Aetzen mit Säure oder im Plasma, Dotieren oder Beschichten, zeigen sie hervorragende Eigenschaften und gewährleisten einen wirksamen Schutz der mit den Fotoresist-Reliefstrukturen abgedeckten Bereiche des Schichtträgers.

### Beispiele:

### Herstellung von Naphthochinondiaziden

Beispiel 1: 12,3 g 2,3,4,4'-Tetrahydroxybenzophenon und 40,3 g 1,2-Naphthochinon-2-diazid-5-sulfonylchlorid werden in 300 ml γ-Butyrolacton gelöst. 15,2 g Triethylamin, gelöst in 50 ml Aceton, werden dann unter Rühren in die vorgelegte Lösung zugetropft. Die Temperatur wird während der Reaktion auf 25°C gehalten, und das Zutropfen ist nach 30 Minuten beendet. Danach wird 3 Stunden nachgerührt, und die Reaktionsmischung wird sodann in 1500 ml 1%-iger HCl gegeben. Der entstandene Niederschlag wird abfiltriert, mit Wasser gewaschen und getrocknet. Die Ausbeute beträgt 39,7 g des erhaltenen Veresterungsprodukts, das aus einem Gemisch von Mono- und Tetraester sowie verschiedenen Isomeren der Di- und Triester besteht.

Beispiel 2: 10 g 2,4,6,3',5'-Biphenylpentol (Pentahydroxybiphenyl) und 40,2 g 1,2-Naphthochinon-2-diazid-5-sulfonylchlorid werden in 500 ml Aceton gelöst. Eine Lösung von 15,2 g Triethylamin in 50 ml Aceton wird während 30 Minuten zugetropft. Es wird 3 Stunden bei 25°C nachgerührt. Nach Fällen des Produkts in 2,5 Liter 1%-iger HCl wird der Niederschlag abfiltriert und getrocknet. Man erhält 38,7 g eines Veresterungsprodukts, das aus einem Gemisch von Mono- und Tetraester sowie verschidenen Isomeren der Di-und Triester besteht.

### Herstellung der Novolake

Beispiel 3 (Harz 1): Zu 40 g m-Kresol, 60 g p-Kresol und 49,0 g einer 37%-igen wässrigen Formaldehyd-Lösung in einem 3-Halskolben werden 0,13 g Oxalsäuredihydrat gegeben, dann wird die Mischung 15 Stunden bei 100°C refluxiert. Nach Abkühlen auf Raumtemperatur wird Vakuum angelegt (5 Torr; = 6,665.10²Pa), und die Temperatur wird lagsam auf 200°C erhöht. Dabei destillieren Wasser und restliche Monomere ab. Es wird ein Novolak mit einem mittleren Molekulargewicht (Mw) von 7100 erhalten (= Harz 1).

Beispiel 4 (Harz 2): Zu 60 g m-Kresol, 40 g p-Kresol und 48,6 g einer 37%-igen wässrigen Formaldehydlösung in einem 3-Halskolben werden 0,05 g Oxalsäuredihydrat gegeben; die Mischung wird dann 15 Stunden bei 100°C refluxiert. Nach Abkühlen auf Raumtemperatur wird Vakuum angelegt (30 Torr; = 39,99.10²Pa), und die Temperatur wird auf 150°C erhöht. Dabei destillieren Wasser und restliche Monomere ab. Es wird ein Novolak mit einem mittleren Molekulargewicht von 7350 erhalten. Dieser Novolak wird wie folgt fraktioniert:

30 g des erhaltenen Novolaks werden in einer Mischung aus 15 g Ethylcellosolveacetat und 90 g Methanol gelöst, und 30 g Wasser werden zugegeben und gerührt. Beim Stehenlassen erfolgt dann eine Phasentrennung, und die untere Phase wird abgetrennt. Aus dieser unteren Phase werden restliches Wasser und Methanol durch Vakuumdestillation entfernt. Es wird ein fraktionierter Novolak mit einem mittleren Molekulargewicht von 10250 erhalten (= Harz 2).

### Lithographische Prüfungen

### Formulierungen

Jeweils 5 g einer Chinondiazidverbindung gemäss Beispiel 1 bzw. Beispiel 2,20 g eines Novolaks und die in der Tabelle 1 genannte Verbindung der Formel I [THPP] werden in 75 g Ethylcellosolveacetat gelöst, und die Resistlösungen werden durch einen 0,2 µm-Porenweite Mikrofilter filtriert.

**Tabelle 1**

| Beispiele | Novolak | Chinondiazid-Verb. | THPP (Komponente c) |
|---|---|---|---|
| 5 | Harz 1 | Bspl. 1 | 2 g |
| 6 | Harz 1 | Bspl. 2 | 2 g |
| 7 | Harz 2 | Bspl. 1 | 4 g |
| 8 | Harz 2 | Bspl. 2 | 5 g |
| THPP = 1,3,3,5-Tetra(p-hydroxyphenyl)pentan [gemäss Formel II, worin m und n 2 sind]. | | | |

### Lithographische Ergebnisse

Die in Tabelle 1 genannten Photoresistlösungen werden bei einer Drehzahl von etwa 5000 Min⁻¹ auf Siliziumscheiben (Wafer) aufgeschleudert, so dass nach der Trocknung bei 90°C und 60 Sekunden auf einer Vakuum-Hotplate eine Schichtdicke von 1,23 Mikron resultiert. Die beschichteten Scheiben werden durch eine Auflösungs-Test-Maske mit einem Canon-Linienstepper FPA-1550 belichtet und anschliessend 120 Sekunden lang bei 110°C auf der Hotplate erhitzt (Post-Exposure-Bake). Danach wird im Puddle-Verfahren 60 Sekunden lang mit einer wässrigen, 2,38%-igen Tetramethylammoniumhydroxyd-Lösung entwickelt. Nach Spülen mit Wasser und Trockenschleudern werden die Scheiben im Rasterelektronenmikroskop im Hinblick auf Lichtempfindlichkeit, Auflösung und Entwicklungsrückstände zwischen den Resistlinien hin untersucht, wobei die Mindestbelichtungsenergie (Lichtempfindlichkeit Eo in mJ/cm²) und die Auflösung (in µm) gemessen werden.

Die Ergebnisse sind in Tabelle 2 zusammengefasst.

**Tabelle 2**

| Beispiele | Lichtempfindlichkeit E (mJ/cm²) | Auflösung (µm) | Rückstände |
|---|---|---|---|
| 9 (aus B.5) | 120 | 0,55 | keine |
| 10 (aus B.6) | 105 | 0,55 | keine |
| 11 (aus B.7) | 205 | 0,52 | keine |
| 12 (aus B.8) | 170 | 0,52 | keine |

Im Vergleich zu bekannten aromatischen Hydroxyverbindungen, wie 2,4,6,3',5'-Pentahydroxy-biphenyl oder 2,3,4,3',4',5'-Hexahydroxybenzophenon, zeichnen sich die mit der erfindungsgemäss eingesetzten Verbindung THPP [1,3,3,5-Tetra(p-hydroxyphenyl)pentan] erhaltenen Positiv-Fotoresist-Formulierungen und die daraus erhaltenen Produkte durch eine höhere Auflösung und ohne Bildung von Rückständen aus. Im Vergleich mit einem Resist ohne Zusatz einer Hydroxyverbindung ist die Lichtempfindlichkeit dabei stark verbessert.

### Beispiel 13: Herstellung einer neuen Chinondiazidverbindung aus 1,2-Naphthochinon-2-diazid-5-sulfonsäure und 1,3,3,5-Tetra(p-hydroxyphenyl)pentan:

5,0 g 1,3,3,5-Tetra(p-hydroxyphenyl)pentan (THPP) und 9,16 g 1,2-Naphthochinon-2-diazid-5-sulfonylchlorid werden in 50 ml γ-Butyrolacton gelöst. 3,52 g Triethylamin, gelöst in 40 ml Aceton, werden unter Rühren während 30 Minuten zugetropft. Die Reaktionstemperatur wird auf 25°C gehalten. Es wird 5 Stunden nachgerührt. Anschliessend wird das Produkt in 400 ml 1%-iger HCl gefällt. Der Niederschlag wird abfiltriert, mit Wasser gewaschen und getrocknet, Ausbeute: 11,5 g eines Veresterungsprodukts, das aus einem Gemisch von Mono- und Tetraester sowie verschiedenen Isomeren der Di- und Triester besteht

### Beispiele 14 und 15: Formulierungen (lithographische Prüfungen)

Die geprüften Formulierungen aus jeweils 5 g einer Chinondiazid-Verbindung gemäss Bsp. 1 bzw. Bsp. 2, 20 g eines Novolaks (gemäss Beispiel 3 bzw. 4) und, im Beispiel 15, zusätzlich noch 4 g der Verbindung THPP (gemäss Formel II) sind in Tabelle 3 angegeben.

**Tabelle 3**

| Beispiele | Novolak | THPP | Diazid-Verbindung gemäss |
|---|---|---|---|
| 14 | Harz 1 | ohne | Beispiele 13 |
| 15 | Harz 2 | 4 g | Beispiele 13 |

### Beispiele 16 und 17: Lithographische Ergebnisse:

Die lithographische Prüfung wird gemäss dem Verfahren von Beispielen 9 bis 12 durchgeführt. Zusätzlich wird die Kristallisarionstendenz der jewiligen Naphthochinondiazid-Verbindung gemäss Bsp. 13 in einem beschleunigten Alterungstest nach 30 Tagen bei 40°C bewertet.

Die Ergebnisse sind in Tabelle 4 zusammengefasst.

**Tabelle 4**

| Beispiele | Lichtempfindlichkeit (mJ/cm²) | Auflösung (µm) | Rückstände | Kristallisation |
|---|---|---|---|---|
| 16 (aus B.14) | 155 | 0,55 | keine | keine |
| 17 (aus B.15) | 195 | 0,52 | keine | keine |

Die damit erhaltenen Positiv-Fotoresist-Zusammensetzungen zeichnen sich durch eine sehr hohe Auflösung ohne Kristallisationsneigung nach 30 Tagen Lagerung bei 40°C aus. Im Gegensatz zu den entsprechenden Positiv-Fotoresist-Zusammensetzungen, die das obige Harz 1 und das Veresterungsprodukt gemäss Beispiel 1 oder Beispiel 2 enthalten jedoch ohne die Verwendung von THPP, zeigen die erfindungsgemässen Zusammensetzungen keine Kristallisationsneigung.

### Beispiel 18 (Herstellung von THPP):

5 kg (18,7 Mol) 1,5-Bis-(p-hxdroxyphenylpenta--1,4-dien-3-on, das durch Kondensation von 2 Mol p-Hydroxybenzaldehyd mit einem Mol Aceton unter Säurekatalyse erhalten wurde, werden in 32 Litern DMF (Dimethylformamid) gelöst und mit 250 g RaneyNi (ethanolfeucht) als Katalysator bei Raumtemperatur mit Wasserstoff bei einem Druck von 5 bar (= 5.10⁵ Pa) hydriert. Die Wasserstoffaufnahme beträgt 841,16 Liter (Dauer der Hydrierung 2,5 Stunden). Nach dem Abdestillieren des Lösungsmittels verbleiben 5 kg eines braunen, viskosen Oels [1,5-Bis(p-hydroxyphenyl)pentan-3-on], das direkt für die nächste Stufe eingesetzt wird.
1015,5 g (3,76 Mol) des so erhaltenen Zwischenproduktes [1,5-Bis(p-hydroxyphenyl)pentan-3-on], 1767,7 g (24,1 Mol) Phenol und 26,3 ml 3-Mercapto-propionsäure werden in einem 6 L-Reaktionskolben, versehen mit Rührer, Rückflusskühler, Thermometer und Gaseinleitung für HCl-Gas sowie Gasableitung mit nachgeschalteter HCl-Absorptionsanlage, vorgelegt und mittels eines thermostatisierbaren Oelbades auf 60°C Innentemperatur erwärmt. Während 1 Stunde wird HCl-Gas in leichtem Strom eingeleitet, und die Temperatur wird bei 70°C gehalten. Dann rührt man während 3 Stunden bei dieser Temperatur ohne HCl-Einleitung. Diese Sequenz von 1 Stunde Gaseinleitung und 3 Stunden Rühren wird dreimal wiederholt Dann gibt man weitere 500 g Phenol dazu und wiederholt die Sequenz noch zehnmal. Dies ergibt eine totale Reaktionszeit von 56 Stunden. Die dunkelviolette viskose Reaktionsmasse wird mit 2000 ml geschmolzenem Phenol verdünnt, und das Festprodukt wird abfiltriert. Der feste Filterrückstand wird in 3000 ml heissem Wasser suspendiert, filtriert und mit heissem Wasser gut nachgespült. Nach Trocknung des Rückstandes im Vakuum bei 120°C während 24 Stunden erhält man 1451,4 g (= 87,6 % Ausbeute, bezogen auf Keton) weiss-violette Kristalle des 1,3,3,5-Tetra(p-hydroxyphenyl)pentans.
Schmelzpunkt: 252°C (mit DSC gemessen); Produkt in Aceton gut löslich.
Phenolische HO-Gruppen: 9,469 Mol/kg (theoretisch 9,080 Mol/kg);

| | | | |
|---|---|---|---|
| Elementaranalyse | Berechnet | C 79,07 % | H 6,41 %; |
| | Gefunden | C 77,96 % | H 6,45 %. |

Beipsiel 19: [1,5-Di(parahydroxyphenyl)-3,3-di(4-hydroxy-3-methylphenyl)-pentan]: 27,00 g (0,10 Mol) des Zwischenprodukts 1,5-Bis(parahydroxyphenyl)pentan-3-on gemäss obigem Beispiel 18 (erstem Teil), 108,14 g (1,00 Mol) ortho-Kresol und 1,50 ml 3-Mercapto-propionsäure werden in einem 750 ml-Reakrionskolben gemäss Beispiel 18 vorgelegt und auf 60°C Innentemperatur erwärmt. Gemäss der Vorschrift von Beispiel 18 leitet man während 48 Std. HCl-Gas aus der Bombe in periodischen Abständen ein. Die Reaktion lässt sich mittels Dünnschichtchromatographie auf Silicagelplatten (Laufmittel = Methylenchlorid:Methanol 95:5) verfolgen. Am Schluss der Reaktion ist kein Edukt mehr sichtbar.

Das Reaktionsprodukt wird in Ethylacetat aufgenommen und mit einer wässrigen, gesättigten Natrium-Bicarbonatlösung bis zur neutralen Reaktion ausgeschüttelt. Nach dem Trocknen der organischen Phase über Magnesiumsulfat filtriert man und entfernt das Lösungsmittel am Rotationsverdampfer. Der Überschuss an ortho-Kresol wird durch Destillation im Hochvakuum entfernt. Es verbleiben 60,68 g eines dunklen Rohprodukts, das zuerst in Toluol verrührt wird, und das Festprodukt wird dann in Methylenchlorid aufgenommen, filtriert und getrocknet im Vakuum bei 50°C. Es resultieren 29,85 g (63,7 % d. Th.) 1,5-Bis(parahydroxyphenyl)-3,3-bis(4-hydroxy-3-methylphenyl)pentan als orange gefärbte Kristalle. Zu dessen Charakterisierung wird eine Probe dreimal aus Methanol/Wasser umkristallisiert, worauf farblose Kristalle erhalten werden.

Smp.: 215,7°C (mit DSC gemessen);

| | | | |
|---|---|---|---|
| Elementaranalyse | berechnet | C 79,46; | H 6,88; |
| | gefunden | C 78,04; | H 6,91. |

Beispiel 20: (Verwendung von THPP gemäss Beispiel 18 als Härter) Ein flammgehemmtes Epoxidharz (Reaktionsprodukt aus Bisphenol-A-diglycidylether und dem entsprechenden bromierten Bisphenol-A)(Epoxygehalt : 2-Aeq/kg; FR 4-Harz) wird mit THPP als Härter und 2-Methylimidazol als Beschleuniger nach üblichem Verfahren gehärtet und daraus Laminate hergestellt.
- Verwendete Harzmenge:: 25 Gew.%, bezogen auf 100 % Festharz.
- Äquivalenzverhältnisse:: Epoxidharz zu THPP zu 2-Methylimidazol: 1,0 Mol zu 0,25 Mol zu 0,0032 Gew%.

Die Glasübergangstemperatur Tg der erhaltenen Laminate beträgt 133°C, und die N-Methylpyrrolidonaufnahme (in %) ist 0,50. Die Laminate zeichnen sich durch eine niedrige Wasser- und NMP-Aufnahme.

### Beispiel 21: 1,3,3,5-Tetra(p-glycidyloxyphenyl)-pentan

313,0 g (0,71 Mol) des Tetraphenols THPP (gemässs Beispiel 18), 1577,9 g (17,05 Mol) Epichlorhydrin und 770 ml 1-Methoxy-2-propanol werden in einem 4,5 1 Reaktionskolben, versehen mit Rührer, Thermometer, Tropftrichter und Heizbad, vorgelegt und auf 50°C erwärmt. Unter gutem Rühren werden 233 g (2,91 Mol) einer 50 %igen wässrigen Natriumhydroxydlösung innert 2,5 h derart zugetropft, dass die Temperatur nicht über 60°C steigt (gelegentliche Kühlung mit Eiswasser erforderlich). Man rührt die Suspension noch 2 h bei 50°-60°C, gibt wenig Trockeneis dazu (zur Neutralisation eines eventuellen Basenüberschusses) und entfernt überschüssiges Epichlorhydrin und Lösungsmittel durch Destillation bei vermindertem Druck (80-130 mbar). Zum noch warmen Destillationsrückstand fügt man 500 ml Isobutylmethylketon bei, rührt die Suspension 0,5 h bei Raumtemperatur und filtriert. Das Filtrat wird am Rotationsverdampfer zur Trockne abrotiert, und der verbleibende harzartige, braune Rückstand (529,7 g) wird in einer Kurzwegdestillationsapparatur bei 150°C Manteltemperatur und einem Vakuum von 0,01 mbar von flüchtigen Anteilen befreit. Man erhält 361,7 g (76% der Th., bezogen auf Tetraphenol THPP) eines festen, braunen Harzes, das 1,3,3,5-Tetra(p-glycidyloxyphenyl)-pentan ist.
Charakterisierung:
- Epoxidwert:: 5,5 Mol/kg (91 % d. Th.);
- Viskosität:: 640 mPa.s bei 120°C;
- GPC (Polystyroleichung):: Mw = 809; Mn = 733; Mw/Mn = 1,10

Beispiel 22: Durch Mischen von 21 Gew.% des Produktes gemäss Beispiel 21 mit einem Reaktionsprodukt aus Bisphenol-A-diglycidylether und dem entsprechenden Tetrabrombisphenol-A (sogenanntes FR 4-Harz; Epoxygehalt 2 Aeq/kg) wird durch Zugabe eines Härtungsmittel-Beschleuniger-Gemisches (Dicyandiamid/2-Methylimidazol; 3,5 Gew.% Dicyandiamid und 0,04 Gew.% 2-Methylimidazol, bezogen auf 100 Gew. Teile Festharz) nach üblichem Verfahren ein Laminat (Presstemp. 170°C; Pressdauer: 90 Min.) mit einem hohen Tg von 160°C und einer NMP-Aufnahme von 0% erhalten.

## Patentansprüche

1. Positiv-Fotoresist-Zusammensetzungen, enthaltend in einem organischen Lösungsmittel mindestens je
a) ein alkalilösliches Harz,
b) eine fotoempfindliche Chinondiazid-Verbindung,
c) eine aromatische Hydroxyverbindung der Formel I 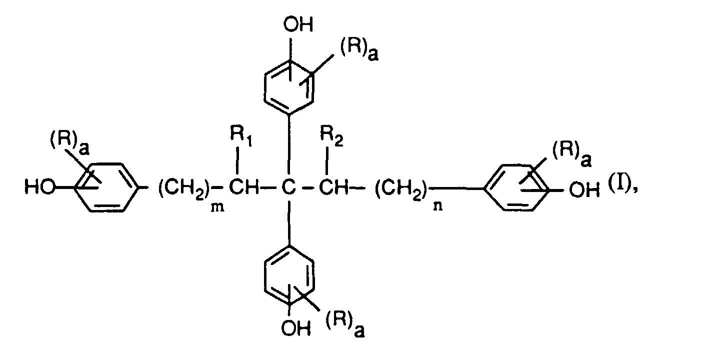 worin R unabhängig voneinander -H, C₁-C₄-Alkyl, C₁-C₄-Alkoxy, -OCH₂C₆H₅, -OC₆H₅ oder -COOC₁-C₄-Alkyl, und R₁ und R₂ unabhängig voneinander H, C₁-C₄-Alkyl, -C₆H₅ oder einen cycloaliphatischen 5- oder 6-Ring bedeuten, a eine ganze Zahl von Null bis 4, und m und n unabhängig voneinander die Zahl Null, 1 oder 2 darstellen, sowie gegebenenfalls
d) weitere übliche Zusätze.

2. Positiv-Fotoresist-Zusammensetzungen nach Anspruch 1, worin in der Formel I R₁ und R₂ -H bedeuten, und m und n die Zahl 1 oder 2 darstellen.

3. Positiv-Fotoresist-Zusammensetzungen nach Anspruch 1, worin als Komponente c) eine Verbindung der Formel II verwendet wird 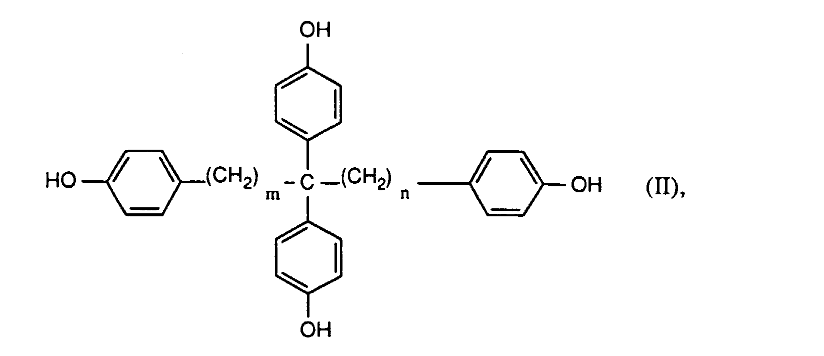 worin m und n unabhängig voneinander die Zahl 1, 2 oder 3 sind.

4. Positiv-Fotoresist-Zusammensetzungen nach Anspruch 3, dadurch gekennzeichnet, dass m und n die Zahl 2 sind.

5. Positiv-Fotoresist-Zusammensetzungen nach Anspruch 1, dadurch gekennzeichnet, dass darin die Verbindung der Formel I in einem Anteil von 0,5-30 Gew.%, bezogen auf den Gesamtfeststoffgehalt der Fotoresistlösung, enthalten ist.

6. Positiv-Fotoresist-Zusammensetzungen nach Anspruch 1, worin die Komponente a) ein Novolak-Harz ist.

7. Positiv-Fotoresist-Zusammensetzungen nach Anspruch 6, worin das Novolak-Harz ein Kresol-Formaldehyd-Harz ist.

8. Positiv-Fotoresist-Zusammensetzungen nach Anspruch 1, worin die Komponente b) ein Veresterungsprodukt der 1,2-Naphthochinon-2-diazid-5-sulfonsäure oder 1,2-Naphthochinon-2-diazid-4-sulfonsäure mit niedermolekularen aromatischen oder aliphatischen Hydroxyverbindungen ist.

9. Positiv-Fotoresist-Zusammensetzungen nach Anspruch 8, worin das Veresterungsprodukt ein Ester der 1,2-Naphthochinon-2-diazid-5-sulfonsäure mit einem mehrfach hydroxylierten Benzophenon oder Biphenyl ist.

10. Positiv-Fotoresist-Zusammensetzungen nach Anspruch 1 enthaltend
50-90 Gew.% der Komponente a),
5-30 Gew.% der Komponente b),
5-20 Gew.% der Komponente c), und gegebenenfalls
0-30 Gew.% an sonstigen Zusatzstoffen,
bezogen auf den Gesamtfeststoffgehalt.

11. Verwendung der Verbindungen der Formel I nach Anspruch 1 als Additive zur Steigerung der Fotoempfindlichkeit und/oder der Entwicklungsrate in Positiv-Fotoresist-Zusammensetzungen auf Basis alkalilöslicher Harze und lichtempfindlicher Chinondiazid-Verbindungen.

12. Die aus den Positiv-Fotoresist-Zusammensetzungen gemäss Anspruch 1 hergestellten Produkte.

13. Veresterungsprodukte der 1,2-Naphthochinon-2-diazid-4- oder -5-sulfonsäure oder 2,1-Naphthochinon-1-diazid-4- oder -5-sulfonsäure mit einer Verbindung der Formel I 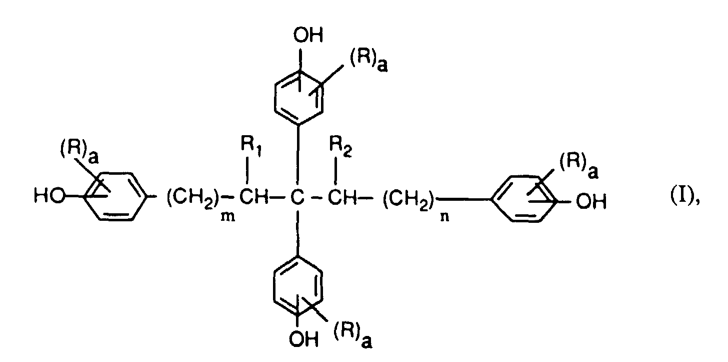 worin R unabhängig voneinander -H, C₁-C₄-Alkyl, C₁-C₄-Alkoxy, -OCH₂C₆H₅, -OC₆H₅ oder -COOC₁-C₄-Alkyl, und R₁ und R₂ unabhängig voneinander H, C₁-C₄-Alkyl, -C₆H₅ oder einen cycloaliphatischen 5- oder 6-Ring bedeuten, a eine ganze Zahl von Null bis 4, und m und n unabhängig voneinander die Zahl Null, 1 oder 2 darstellen.

14. Veresterungsprodukte gemäss Anspruch 13 der Formel II, worin als Verbindung der Formel I eine Verbindung der Formel II 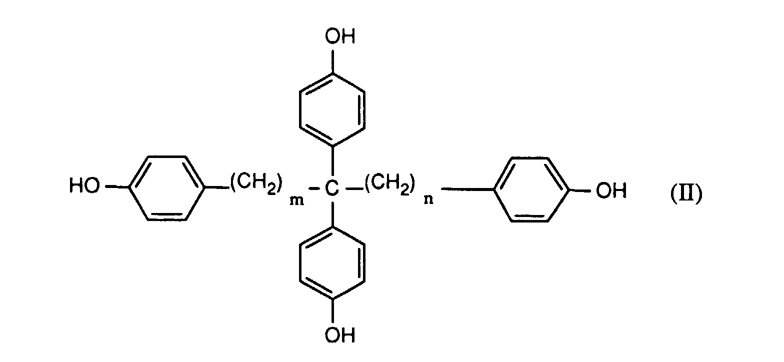 eingesetzt wird, worin m und n unabhängig voneinander die Zahl 1, 2 oder 3 darstellen.

15. Verwendung der Veresterungsprodukte gemäss Anspruch 13 als fotoaktive Komponente in Positiv-Fotoresist-Formulierungen.

16. Tetra(hydroxyphenyl)alkane der Formel (I) 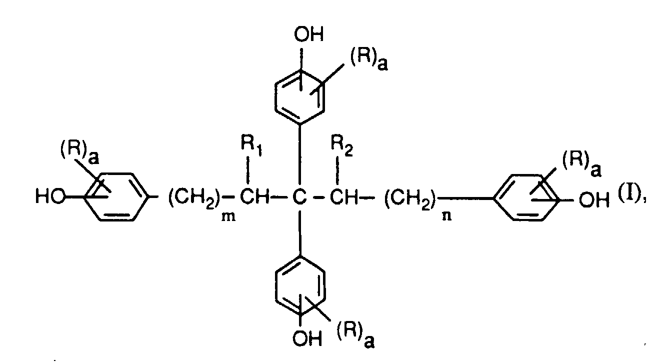 worin R unabhängig voneinander -H, C₁-C₄-Alkyl, C₁-C₄-Alkoxy, -OCH₂C₆H₅, -OC₆H₅ oder -COOC₁-C₄-Alkyl, und R₁ und R₂ unabhängig voneinander H, C₁-C₄-Alkyl, -C₆H₅ oder einen cycloaliphatischen 5- oder 6-Ring bedeuten, a eine ganze Zahl von Null bis 4, und m und n unabhängig voneinander die Zahl Null, 1 oder 2 darstellen, wobei, wenn R₁ und R₂ -H und a Null oder 1 bedeuten, m und n die Zahl Null nicht darstellen dürfen.

## Claims

1. Positive photoresist compositions, containing in an organic solvent at least one each of:
a) an alkali-soluble resin,
b) a photosensitive quinone diazide compound,
c) an aromatic hydroxy compound of formula I 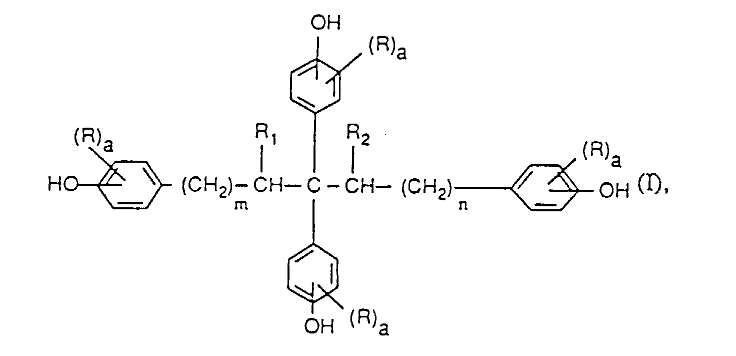 in which R independently of one another denotes -H, C₁-C₄ alkyl, C₁-C₄ alkoxy, -OCH₂C₆H₅, -OC₆H₅ or -COOC₁-C₄ alkyl, and R₁ and R₂ independently of one another denote H, C₁-C₄ alkyl, -C₆H₅ or a cycloaliphatic 5-membered or 6-membered ring, a represents an integer fron zero to 4, and m and n independently of one another represent the number zero, 1 or 2,
as well as, if appropriate,
d) further standard additives.

2. Positive photoresist compositions according to claim 1, in which in formula I R₁ and R₂ denote -H, and m and n are the number 1 or 2.

3. Positive photoresist compositions according to claim 1, in which as component c) a compound of formula II is used 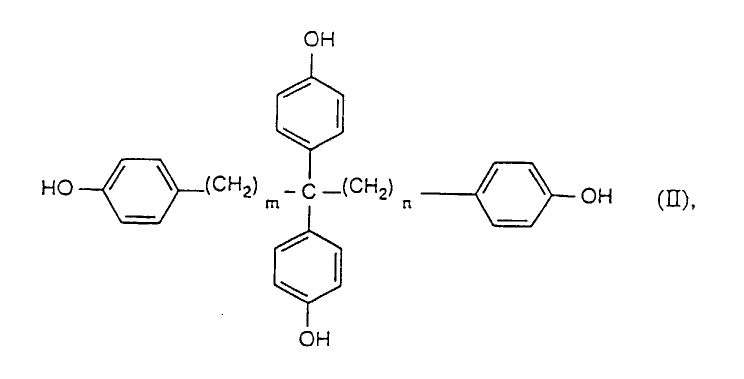 in which m and n independently of one another are the number 1, 2 or 3.

4. Positive photoresist compositions according to claim 3, characterised in that m and n are the number 2.

5. Positive photoresist compositions according to claim 1, characterised in that the compound of formula I is contained therein in a proportion of 0.5 - 30 % by weight, based on the total solids content of the photoresist solution.

6. Positive photoresist compositions according to claim 1, in which component a) is a novolak resin.

7. Positive photoresist compositions according to claim 6, in which the novolak resin is a cresol formaldehyde resin.

8. Positive photoresist compositions according to claim 1, in which component b) is an esterification product of 1,2-naphthoquinone-2-diazido-5-sulphonic acid or 1,2-naphthoquinone-2-diazido-4-sulphonic acid with lower molecular aromatic or aliphatic hydroxy compounds.

9. Positive photoresist compositions according to claim 8, in which the esterification product is an ester of 1,2-naphthoquinone-2-diazido-5-sulphonic acid with a polyhydroxylated benzophenone or biphenyl.

10. Positive photoresist compositions according to claim 1, containing 50 to 90 % by weight of component a),
5 to 30 % by weight of component b),
5 to 20 % by weight of component c), and if appropriate
0 to 30 % by weight of other additives,
based on the total solids ccntent.

11. Use of the compounds of formula I according to claim 1 as additives for enhancing the photosensitivity and/or development rate in positive photoresist compositions based on alkali-soluble resins and light-sensitive quinone diazide compounds.

12. The products made from the positive photoresist compositions in accordance with claim 1.

13. Esterification products of
1,2-naphthoquinone-2-diazido-4-sulphonic acid or
1,2-naphthoquinone-2-diazido-5-sulphonic acid or
2,1-naphthoquinone-1-diazido-4-sulphonic acid or
2,1-naphthoquinone-1-diazido-5-sulphonic acid with a compound of formula I 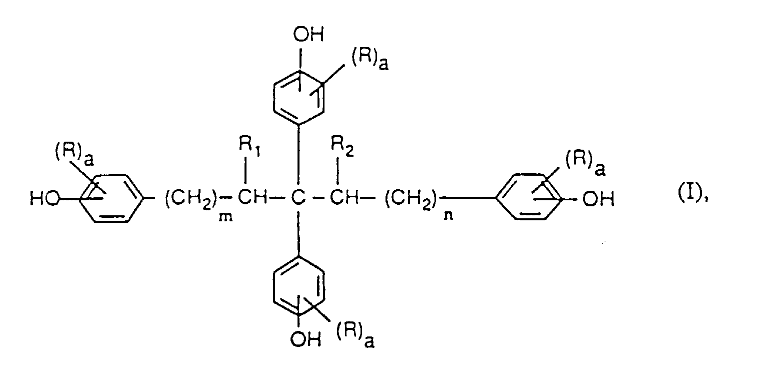 in which R independently of one another denotes -H, C₁-C₄ alkyl, C₁-C₄ alkoxy, -OCH₂C₆H₅, -OC₆H₅ or -COOC₁-C₄ alkyl, and R₁ and R₂ independently of one another denote H, C₁-C₄ alkyl, -C₆H₅ or a cycloaliphatic 5-membered or 6-membered ring, a represents an integer from zero to 4, and m and n independently of one another represent the number zero, 1 or 2.

14. Esterification products in accordance with claim 13 of formula II, in which as the compound of formula I a compound of formula II 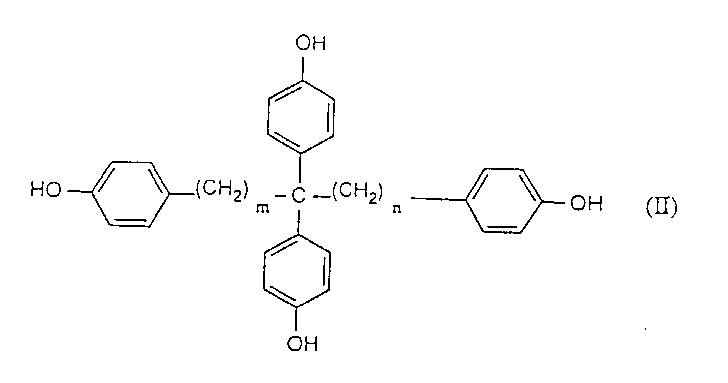 is used, in which m and n independently of one another represent the number 1, 2 or 3.

15. Use of the esterification products in accordance with claim 13 as the photoactive component in positive photoresist formulations.

16. Tetra(hydroxyphenyl)alkanes of formula I 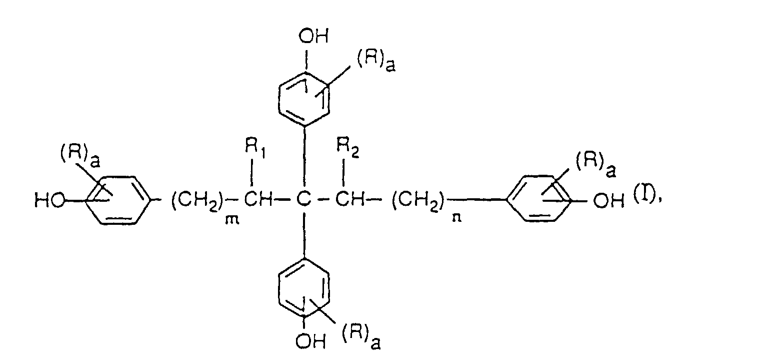 in which R independently of one another denotes -H, C₁-C₄ alkyl, C₁-C₄ alkoxy, -OCH₂C₆H₅, -OC₆H₅ or -COOC₁-C₄ alkyl, and R₁ and R₂ independently of one another denote H, C₁-C₄ alkyl, -C₆H₅ or a cycloaliphatic 5-membered or 6-membered ring, a represents an integer from zero to 4, and m and n independently of one another represent the number zero, 1 or 2, with the proviso that if R₁ and R₂ are -H and a denotes zero or 1, m and n cannot be zero.

## Revendications

1. Compositions de photorésists positifs contenant dans un solvant organique chacune au moins
a) une résine soluble en milieu alcalin,
b) un composé de quinonediazide photosensible,
c) un composé hydroxylé aromatique de formule I 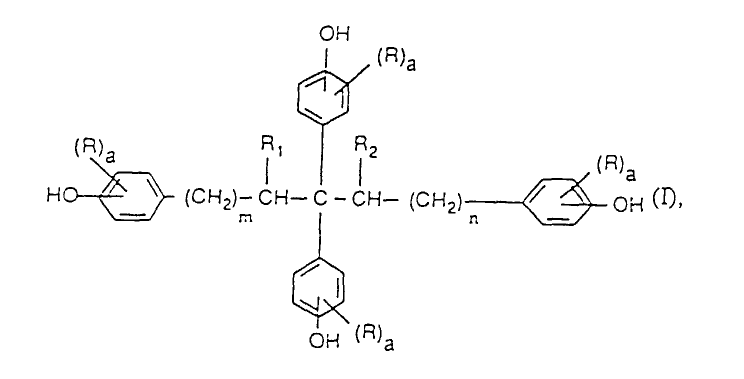 où les R représentent indépendamment les uns des autres -H, alkyle en C₁-C₄, alcoxy en C₁-C₄, -OCH₂C₆H₅, -OC₆H₅ ou -COO-alkyle en C₁-C₄, et R₁ et R₂ représentent indépendamment l'un de l'autre H, alkyle en C₁-C₄, -C₆H₅ ou un cycle cycloaliphatique à 5 ou 6 chaînons, a représente un nombre entier de 0 à 4 et m et n représentent indépendamment l'un de l'autre le nombre 0, 1 ou 2, et éventuellement
d) d'autres additifs courants.

2. Compositions de photorésists positifs selon la revendication 1, où dans la formule I R₁ et R₂ représentent -H et m et n représentent le nombre 1 ou 2.

3. Compositions de photorésists positifs selon la revendication 1, où comme composant c) on utilise un composé de formule II 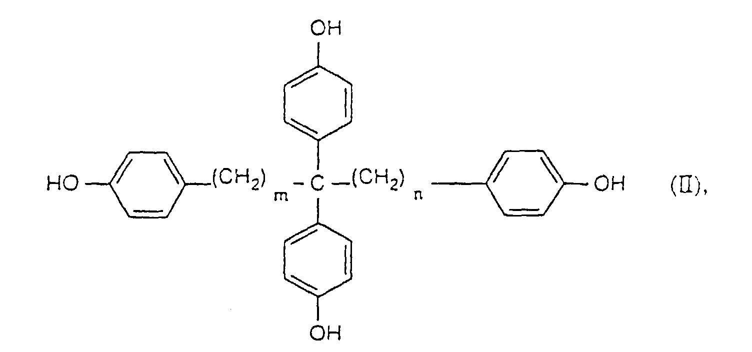 où m et n sont indépendamment l'un de l'autre le nombre 1, 2 ou 3.

4. Compositions de photorésists positifs selon la revendication 3, caractérisées en ce que m et n sont le nombre 2.

5. Compositions de photorésists positifs selon la revendication 1, caractérisées en ce que le composé de formule I est contenu en une proportion de 0,5-30 % en masse par rapport à la teneur en solides totale de la solution de photorésist.

6. Compositions de photorésists positifs selon la revendication 1, où le composant a) est une résine novolaque.

7. Compositions de photorésists positifs selon la revendication 6, où la résine novolaque est une résine crésol-formaldéhyde.

8. Compositions de photorésists positifs selon la revendication 1, où le composé b) est un produit d'estérification de l'acide 1,2-naphtoquinone-2-diazide-5-sulfonique ou de l'acide 1,2-naphtoquinone-2-diazide-4-sulfonique avec des composés hydroxylés aromatiques ou aliphatiques de faible masse moléculaire.

9. Compositions de photorésists positifs selon la revendication 8, où le produit d'estérification est un ester de l'acide 1,2-naphtoquinone-2-diazide-5-sulfonique avec une benzophénone hydroxylée plusieurs fois.

10. Compositions de photorésists positifs selon la revendication 1, contenant
50-90 % en masse de composant a),
5-30 % en masse de composant b),
5-20 % en masse de composant c), et éventuellement
0-30 % en masse d'autres additifs,
par rapport à la teneur totale en solides.

11. Utilisation des composés de formule I selon la revendication 1 comme additifs pour augmenter la photosensibilité et/ou la vitesse de développement dans des compositions de photorésists positifs à base de résines solubles en milieu alcalin et de composés de quinonediazide sensibles à la lumière.

12. Produits fabriqués à partir des compositions de photorésists positifs selon la revendication 1.

13. Produits d'estérification de l'acide 1,2-naphtoquinone-2-diazide-4- ou 5-sulfonique ou de l'acide 2,1-naphtoquinone-1-diazide-4- ou 5-sulfonique avec un composé de formule I 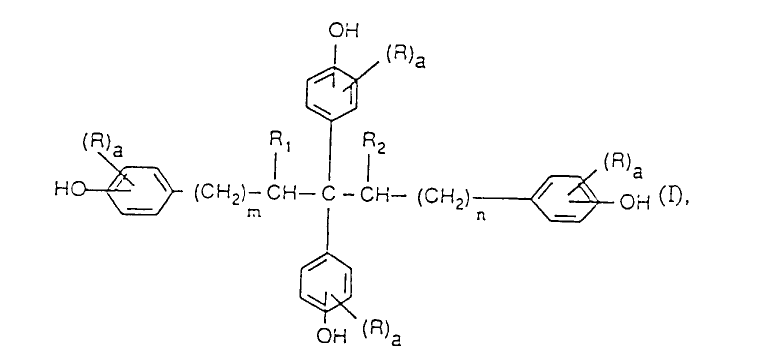 où les R représentent indépendamment les uns des autres -H, alkyle en C₁-C₄, alcoxy en C₁-C₄, -OCH₂C₆H₅, -OC₆H₅ ou -COO-alkyle en C₁-C₄, et R₁ et R₂ représentent indépendamment l'un de l'autre H, alkyle en C₁-C₄, -C₆H₅ ou un cycle cycloaliphatique à 5 ou 6 chaînons, a représente un nombre entier de 0 à 4 et m et n représentent indépendamment l'un de l'autre le nombre 0, 1 ou 2.

14. Produits d'estérification selon la revendication 13 de formule II, ou comme composé de formule I on utilise un composé de formule II 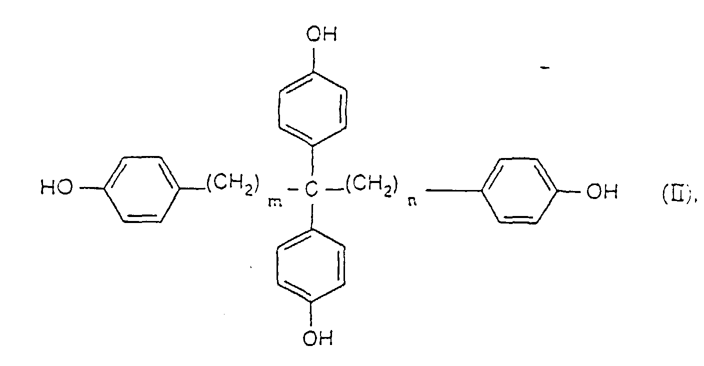 où m et n représentent indépendamment l'un de l'autre le nombre 1, 2 ou 3.

15. Utilisation des produits d'estérification selon la revendication 13 comme composant photoactif dans des formulations de photorésists positifs.

16. Tétra(hydroxyphényl)alcanes de formule I 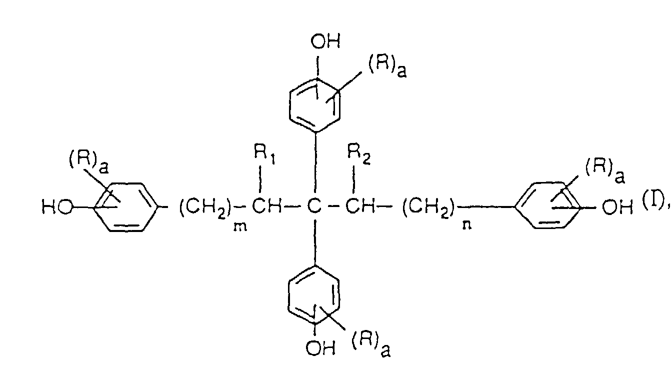 où les R représentent indépendamment les uns des autres -H, alkyle en C₁-C₄, alcoxy en C₁-C₄, -OCH₂C₆H₅, -OC₆H₅ ou -COO-alkyle en C₁-C₄, et R₁ et R₂ représentent indépendamment l'un de l'autre H, alkyle en C₁-C₄, -C₆H₅ ou un cycle cycloaliphatique à 5 ou 6 chaînons, a représente un nombre entier de 0 à 4 et m et n représentent indépendamment l'un de l'autre le nombre 0, 1 ou 2, où, lorsque R₁ et R₂ représentent -H et a représente 0 ou 1, m et n ne doivent pas représenter le nombre 0.
